# EUROPEAN PATENT APPLICATION

(11) **EP 4 717 757 A1**
(43) Date of publication of application: **01.04.2026**
(21) Application number: 24300004.9
(22) Date of filing: 27.09.2024
(51) Int. Cl.: C12M 1/00

(54) **VESSEL AND REACTOR FOR UPSTREAM PRODUCTION OF BIOLOGIC PRODUCTS**

(71) Applicant: Good Dog Food Limited, London EC4M 7AN (GB)
(72) Inventor: DELBRIDGE, Jordan, London EC4M 7AN (GB); BARBA-CARBALLO, Desiree, London EC4M 7AN (GB); CIUBOTAR, Miruna, London EC4M 7AN (GB); LOPES, Magno, London EC4M 7AN (GB); CRUZ, Helder, London EC4M 7AN (GB)
(74) Representative: Keltie LLP

(57) **Abstract**

The present invention relates to vessels for use as bioreactor, and reactor systems or apparatuses including such vessels. The vessels comprise a tank comprising a plastic or first cured resin material which is biocompatible and which can withstand steam sterilization.

## Description

### Field of the Invention

The present invention relates to vessels for use as bioreactor, and reactor systems or apparatuses including such vessels.

(Herein, the word bioreactor is used as an overarching term to include all types and styles of such reactor, such as bioreactors used for, for example, animal cell culturing and fermenters used for bacterial or fungal cell culturing and fermentation.)

### Background

In upstream production of biologic products, the equipment is costly due to the materials used (to allow sterilization and aseptic use) and the number and complexity of the peripherals (pumps, valves, etc).

When the final products have extremely high added value, such as biopharmaceuticals, this is not a problem since the companies developing these products are not cost-sensitive and can absorb high equipment costs.

But when the products need to be competitive in price, such as in the case of cultivated meat, all the cost contributors are relevant; among those are the capital cost of the equipment, which is traditionally very high. A careful cost analysis leads to the conclusion that, although some cost reduction is possible by simplifying the design of the equipment including the peripherals, a very significant cost reduction is necessary to make cultivated meat economically viable. Therefore, the need, particularly in cultivated meat, for novel, more affordable and sustainable vessels is very significant.

The present invention has been devised in the light of the above considerations.

### Summary of the Invention

At its broadest, the present invention relates to vessels which comprise a steam sterilizable and biocompatible plastic or cured resin material. For extra mechanical strength, the vessels may comprise an additional outer layer. At least one of the layers, generally the outer layer, is a composite material comprising a cured resin and reinforcing fibres.

Such vessels have never been used in bioreactors before, and certainly not at industrial scale. The present inventors have found, surprisingly, that such vessels are suitable for repeated sterilization and have high biocompatibility, while offering similar strength to existing technologies and materials. The cost is significantly lower (lower capital cost, transportation and installation costs; lower lifetime cost; reduced operating costs, associated with insulating properties and reduced heating demands) and sustainability (in particular carbon footprint) is significantly improved.

The present vessels offer many other advantages as bioreactors, for example, significant weight reduction (plastics, resins and composites are significantly lighter than metals, while maintaining the mechanical strength to undergo high pressure; reduced weight allows easier transport and installation); corrosion resistance (unlike metals, plastics, resins and composites do not corrode, reducing maintenance costs and increasing the lifespan); and ready availability.

The vessels can be used in any shape or type of bioreactor or fermenter, such as but not limited to a stirred tank, externally agitated tank (e.g. orbitally shaken bioreactor), packed bed, fluidised bed, or airlift, and operated in any mode of operation such as but not limited to batch, repeated batch, fed-batch or continuous with cell retention (perfusion) or without cell retention (chemostat).

The vessel can be used to culture any type of cell, such as but not limited to animal cells, bacteria, yeast, filamentous fungi, algae or plant cells for several applications, including for biopharma, precision fermentation, and cultivated meat or cellular agriculture.

Accordingly, the present invention provides, in a first aspect, the use of a vessel, comprising a rigid tank comprising a plastic or first cured resin material which is biocompatible and which can withstand steam sterilization, as a bioreactor.

In certain embodiments, the vessel further comprises an outer shell of reinforcing fibres impregnated with a second cured resin, the outer shell at least partially encompassing the tank. This outer shell allows increased mechanical strength to accommodate high pressures and represents an alternative to increasing wall thickness of the vessel.

Accordingly, the present invention relates to the use of a vessel, comprising an inner tank of a plastic or first cured resin and an outer shell of reinforcing fibres impregnated with a second cured resin, as a bioreactor.

When the tank herein is mentioned as being 'rigid' what is meant is that it is structurally self-supporting. That is, when placed on a surface the tank does not deform significantly or indeed at all under its own weight. This is to differentiate from plastic bags or the like which deform unless supported by some secondary structure.

For completeness we note that, where the present invention includes the outer shell, that can act as a supporting structure and so the tank need not be rigid in such circumstances, or its thickness may be reduced.

A tank or material is considered as being able to be steam sterilised without deformation (that is, capable of withstanding sterilization) if its structural shape and/or integrity are neither changed nor compromised by steam sterilization, for example in an autoclave or with steam-in-place procedures at larger scales. This differentiates from materials which would melt or deform significantly under such conditions. Steam sterilization is typically carried out at a minimum temperature of 121 °C with a hold of at least 30 minutes at a pressure of up to 220 kPa. A vessel which can undergo steam sterilization under these conditions without structural change, damage, deformation or loss of integrity can be said to be able to withstand steam sterilization.

A material is considered biocompatible herein if it can be in contact with the cell culture medium and cells without adverse effects; for example, without toxicity to the culture, i.e. allowing normal cell growth, viability, metabolism, and morphology to happen throughout a culture.

In some embodiments, wherein the (rigid) tank is formed from a material (plastic or first cured resin) selected from polyethylene, polystyrene, polycarbonate, polypropylene, polyvinyl chloride and polyurethane; preferably selected from polyethylene and polypropylene; more preferably polyethylene for example high density polyethylene.

In some embodiments, the reinforcing fibres, where present, are woven.

In some embodiments, the reinforcing fibres, where present, are preimpregnated with a resin which is then cured to form the outer shell (second cured resin).

The second cured resin may be an epoxy resin, a phenolic resin, a bismaleimide resin, or a polyimide resin; preferably the second cured resin is an epoxy resin.

The reinforcing fibres may suitably be carbon fibres, fibreglass fibres or aramid fibres.

Combinations of these materials have been found to form vessels which are particularly effective as bioreactors.

In some embodiments, the vessel has an internal volume of 3 L or more. The present invention enables a novel, not previously utilized, type of large scale bioreactors of a reduced cost and weight compared to stainless steel while having adequate mechanical strength for the proposed use.

The vessel may have baffles positioned within the tank, optionally attached to the walls of the tank. Such baffles can assist in improving mixing efficiency and dispersing the medium within the vessel, leading to advantageous mixing and cell culture growth.

In some embodiments of the first aspect of the invention, the use is as a stirred tank, externally agitated tank, packed bed, fluidised bed or airlift bioreactor.

In some embodiments of the first aspect of the invention, the use is as a bioreactor in a batch, fed-batch, continuous with cell retention, or continuous without cell retention mode of operation.

In some embodiments of the first aspect of the invention, the use is as a bioreactor for culturing animal cells, bacteria, yeast, filamentous fungi, algae or plant cells.

A second aspect of the present invention provides a bioreactor apparatus, comprising a vessel, the vessel comprising a rigid tank comprising a plastic or first cured resin material which is biocompatible and which can withstand steam sterilization and optionally further comprising an outer shell of reinforcing fibres impregnated with a second cured resin; the apparatus further comprising at least one of: (a) agitation means, for agitating the content of the vessel; (b) temperature control means, for controlling the temperature of the content of the vessel; (c) fluid introduction means, for introducing fluid, suitably liquid, into the vessel; and (d) sparging means, for introducing gas into the vessel.

Accordingly, the present invention also relates to a bioreactor apparatus, comprising a vessel with a main body having an inner tank of a first cured resin and an outer shell of reinforcing fibres impregnated with a second cured resin; the apparatus further comprising at least one of: (a) agitation means, for agitating the content of the vessel; (b) temperature control means, for controlling the temperature of the content of the vessel; (c) fluid introduction means, for introducing fluid, suitably liquid, into the vessel; and (d) sparging means, for introducing gas into the vessel.

Where present, the agitation means may suitably be a stirrer (if internal, that is, positioned within the vessel) or a rocking and/or rolling and/or centrifugal agitation platform (if external to the vessel).

Where present, the temperature control means may suitably be a heated jacket positioned around the outside of the main body, a double wall water jacket, a coil jacket or an internal heating coil.

Where present, the fluid introduction means may comprise a pipe connected to an aperture in the main body, the pipe having a valve to control the flow of a fluid through the pipe into the main body.

The invention includes the combination of the aspects and preferred features described except where such a combination is clearly impermissible or expressly avoided.

### Summary of the Figures

Embodiments and experiments illustrating the principles of the invention will now be discussed with reference to the accompanying figures in which:
**Fig. 1** is a plot of average viable cell concentration (VCC) and average cell viability over time for Example 1 set out below.
**Fig. 2** is a plot of glucose and lactate concentrations over time for Example 1 set out below.
**Fig. 3** is a series of magnified views of the cell culture produced in Example 1 set out below, at different times.

### Detailed Description of the Invention

Aspects and embodiments of the present invention will now be discussed with reference to the accompanying figures. Further aspects and embodiments will be apparent to those skilled in the art. All documents mentioned in this text are incorporated herein by reference.

### Bioreactors - General Discussion

A bioreactor as a vessel is one in which raw materials under controlled conditions are converted into products by activity of living cells (microorganisms, animal cells, plant cells and stem cells, tissues, algae and fungi) or by cellular components such as enzymes. Herein, we use the terms "vessel" and "tank" to discuss a container in which this activity occurs.

A bioreactor as a device or system is designed to cultivate and grow biological cells, tissues, or organisms under controlled conditions. It serves as a vessel-like apparatus that provides a stable environment for cells to flourish and maintains a steady balance in the biochemical processes carried out by these to create desired substances or cell biomass. Herein, we use the term "bioreactor apparatus" to discuss the broader device, including not only the vessel but also additional peripheral parts, as appropriate.

By carefully controlling factors such as temperature, pH, dissolved oxygen, aeration, agitation, nutrient feeding, and sterility, bioreactors create an environment conducive to the growth and metabolic activity of the cells.

This broad definition is the one worked to in the present application.

Bioreactor and fermenter are similar terms, but in certain circumstances in the art they are used with a distinct difference. The term bioreactor often relates to the cultivation of animal, plant and stem cells, while the term fermenter often relates to the cultivation of bacteria, yeast or filamentous fungi. It would not be a mistake to use the term bioreactor in such cases as well, but in the case of cell cultivation only the term bioreactor is used.

The main difference between the bioreactors used for cell and microorganism cultivation is in the mixing and aeration requirements, as well as vessel height to diameter ratio H/D.

Herein, we do not differentiate between "bioreactors" and "fermenters" in this way.

When selecting materials for bioreactor construction in the present invention, several important properties have been considered. The material should be non-corrosive to ensure the integrity and longevity of the bioreactor. It should be biocompatible, that is, not harm cell growth and metabolism or affect product quality. The material should also be able to withstand the steam sterilization process commonly used in bioreactors. Furthermore, it should be capable of tolerating high pressure and resisting changes in pH. Ideally it should be able to keep temperature constant with less expenditure of energy to heat the cultures.

### The present vessels

The vessel in the present invention, at its base, has a rigid tank which is formed from plastic or cured resin material, which is biocompatible and can be steam sterilized without deformation. It may suitably be a structure having at least two layers: a tank and an outer shell at least partially encompassing (that is, enclosing or surrounding) the tank, for increased mechanical resistance.

The tank is, generally, a plastic or polymeric material (such as a first cured resin). The tank, being exposed to the culture conditions and media, should ideally be relatively durable and rigid, as well as offering biocompatibility (allowing normal cell growth and viability) and limiting cell adhesion to the walls. To that end, a relatively smooth inner surface is advantageous, and so materials which can be formed with such a smooth finish are preferred. Moreover, the materials are suitably stable to steam sterilization, ideally to multiple repetitions of steam sterilization.

Suitable materials include plastic or polymeric materials (including cured resins) such as polyethylene, polypropylene, polystyrene, polycarbonate, polyvinyl chloride and polyurethane, or cured resins. Of these, polyethylenes and polypropylenes are preferred. In some embodiments, the material is polyethylene, polypropylene, polyvinyl chloride or polyurethane. In some embodiments the material is a polyethylene.

In particular, high density polyethylene (HDPE) is suitable as the tank, being very readily available while also providing relative strength and biocompatibility at low cost as well as having good thermal insulation properties. HDPE is often defined by its density; a typical density is 930 to 970 kg/m³.

Simple plastic vessels comprising only a shell (either rigid, like a tank, or flexible, like a bag) of, for example, polycarbonate or polystyrene have been used as small scale bioreactors in the past. However, they had been found to be incompatible with repeated sterilization (steam sterilization by autoclave, for example, leading to degradation and in particular melting of the materials; instead gamma, x-ray, beta or other radiation has been used but cannot be adopted given the need to ship to specialised irradiation facilities that make time spent and costs impractical for repetitive use). Moreover, the mechanical strength of such vessels is very limited, meaning their use at volumes above about 50 L has been thought impossible. Apart from simple plastic vessels, which are limited to lab scale (up to 50 L), single use plastic bags of up to 2000 L volumes have been used industrially. These larger scale single use bags are prohibitively expensive and would still require a costly stainless steel supporting structure. As these bags melt if autoclaved they are also sterilized by radiation and are effectively single use. For the reasons stated above including cost and scalability to tens of thousands of litres, these materials are clearly unsuitable for manufacture of vessels or bioreactors for production of low cost biologics, such as cultivated meat.

Accordingly, such simple vessels have only been used as small scale and single use reactors. This is not conducive to industrial implementation, and cannot provide a cost lowering as needed by industry for low cost products such as cultivated meat products.

The present inventors have found that a vessel having the present construction, and in particular a tank of polyethylene that may or may not have an additional outer shell of fibre-reinforced plastic/polymer (that is, reinforcing fibres pre-impregnated with a resin and then cured) can particularly advantageously solve these problems. With this structure the vessels are shown to have biocompatibility and to be capable of repeated sterilization and hence reusability, apart from being mechanically resistant to allow large industrial volumes of tens of thousands of litres of capacity. They solve the problems previously faced.

The outer shell is, generally, of reinforcing fibres impregnated with a second cured resin. The outer shell provides additional rigidity and strength, allowing for a lower thickness of the tank, if needed. Suitable reinforcing fibres include carbon fibres, fibreglass fibres or aramid fibres. The outer shell may include fibres of one type or alternatively of a mixture of types. For example, all reinforcing fibres may be carbon fibres, or all fibreglass fibres, or all aramid fibres. Alternatively, the reinforcing fibres may be a mix of two or more types, or example two or more types chosen from carbon fibres, fibreglass fibres and aramid fibres.

The resin impregnated into the reinforcing fibres and cured may again be broadly chosen. Common and suitable types of resin include epoxy resins, phenolic resins, bismaleimide resins, and polyimide resins. Most suitably, the resin is an epoxy resin.

Accordingly, the outer shell may suitably be formed of carbon fibre-epoxy composite; fibreglass-epoxy composite, or aramid-epoxy composite. Most suitably, the outer shell is formed from a fibreglass-epoxy composite.

The present vessels may be further adapted to be particularly suited to bioreactor usage. For example, the vessel may have an internal volume of 3 L or more to be useful at an industrial scale; ideally, 10 L or more, 20 L or more, 50 L or more, 100 L or more, 200 L or more, 1000 L or more, 2000 L or more, 10000 L or more, or even 20000 L or more.

### Additional Parts

The vessels described herein may comprise or contain additional parts to further their suitability for use in or for bioreactors. Additional parts may also be connected to or operate with the vessel to provide a suitable basis for a bioreactor apparatus.

Heating and cooling means (that is, temperature control means) can play an important role in maintaining optimal temperature conditions during a culture. For example, vessels may be equipped with an outer jacket. This outer jacket may be heated or cooled; that is, the temperature of the jacket can be controlled to correspondingly control the temperature conditions inside the vessel. Such jackets are particularly suitable for vessels with a capacity of 1000 L or less.

Alternatively or additionally, the vessel may be provided with one or more internal heating coils, which may be operated (for example electrically) to heat the interior volume of the vessel.

Maintaining precise temperature conditions is crucial in bioprocessing (cell culture and fermentation), as it directly influences the growth and metabolic activity of cells. One advantage of the present invention is that the present vessels can retain heat much more effectively than metal bioreactors, due to their heat insulating properties which are generally much different than the heat conductive metal. For example, HDPE has a thermal conductivity of 0.5 W/mK, while stainless steel has a thermal conductivity of 15 W/mK. HDPE is therefore a better insulator than stainless steel as it does not easily conduct heat. HDPE is commonly used in applications where thermal insulation is required, such as piping. The positive implication of this is that the energy necessary to heat and maintain the temperature on the inside of the present vessels and the energy necessary to cool down ambient temperature are significantly lower than if using a metal (such as stainless steel) bioreactor tank. This represents a clear advantage in terms of operating costs.

Stirrers (that is, agitation means) can play an important role in the operation of bioreactors by ensuring the uniform suspension of cells in various nutrient media, promoting efficient interaction between the cells and the nutrients, facilitating optimal growth and metabolic activity. Stirrers generally consist of a vertical shaft connected to a motor and one or more impellers. Impellers are typically composed of blades. The number, shape, and size of the blades can vary depending on the specific requirements of the process, but the most commonly used are: a. Disc Turbines: These impellers consist of a series of flat, disc-shaped blades; b. Variable Pitched Blade Turbine: This type of impeller features blades with a variable pitch angle; c. Marine impellers: commonly used in animal cell culture.

Suitably, the vessels of the present invention include one or more agitation means, particularly one or more stirrers for example impellers.

External agitation means are also envisaged, such as a platform, frame or stand which agitates the vessel itself to hence agitate the vessel's contents. It may, for example, act or be moveable in order to rock, roll or centrifuge the vessel.

Some vessels may include a sparger within the vessel. A sparger can play an important role in ensuring proper dissolution of gas within a vessel by introducing sterile gas, for example air or oxygen. It is a system that helps in promoting the growth and metabolism of cells during culture. There are three main types of spargers: a. Porous Sparger: This type of sparger consists of a porous material, such as sintered metal or ceramic, which allows gas to pass through evenly distributed pores. The porous structure helps in creating fine gas bubbles, which enhances the contact between gas and the medium, leading to efficient gas (for example air or oxygen) transfer; b. Nozzle Sparger: Nozzle spargers utilize a system of nozzles or orifices to release pressurized gas, for example air or oxygen, into the vessel. These nozzles are strategically positioned to ensure proper distribution of gas throughout the medium; c. Combined Sparger-Agitator: In some cases, the sparger is integrated with an agitation means such as a stirrer within the vessel. This combined sparger-agitator design helps in achieving both dissolution of gas and agitation simultaneously. The sparger component introduces gas, such as air or oxygen, while the agitator ensures proper mixing and distribution of the gas bubbles, maximizing gas (for example air or oxygen) transfer.

The present vessels may include, for example, one or more baffles located within the vessel to assist in mixing or agitating the culture or reaction media held within the vessel in use. Such baffles may be, for example, flat plates mounted on the inner wall(s) of the tank of the vessel.

Baffles are important components incorporated into bioreactors to serve multiple purposes, such as preventing the formation of a central vortex and improving mixing and mass transfer within the vessel. By disrupting swirling motion and promoting even distribution of air or oxygen, baffles contribute to efficient mixing, mass transfer, and optimal growth of cells.

The vessels may be provided with one or more feed ports (i.e. openings through which substances can be fed into the vessel). Feed ports play a crucial role in the operation of a bioreactor by allowing the addition of nutrients, acids, alkalis, or other substances necessary for the culture.

The vessels may be provided with one or more sample ports (i.e. openings through which substances can be extracted from the vessel). Sample ports are essential for off-line monitoring, such as cell density and viability, as well as nutrient and metabolite levels.

Accordingly, a or each sample port may be provided with a sampling means, for extracting a sample from the vessel. Such sampling means (or plural means) may (each) be connected to analytical equipment, for example one or more of a cell density monitoring means, cell viability monitoring means, nutrient monitoring means, or metabolite monitoring means. Specific examples include means for monitoring glucose levels in the sample and means for monitoring glutamine levels in the sample.

The vessel may be provided with one or more probes for monitoring culture conditions within the vessel. Online probes enable the user to monitor and, in some instances, control those conditions. For example, one or more of a temperature probe, a dissolved oxygen concentration probe and a pH probe may be provided in the vessel.

Valves play an important role in controlling the flow and movement of fluids and gases in a bioreactor. They are used to regulate the passage of fluids and gases, allowing for optimal operation and precise control of a process. Since bioreactors are usually operated with positive pressure to stop the entry of contaminants, safety valves are suitably used, during both sterilization and operation. Accordingly, the present vessels may comprise one or more valves.

Within the field of bioreactors at industrial scale, the majority of the vessel cleaning and sterilisation processes are now automated with spray jets from nozzles located inside the vessel; these processes are referred to as Cleaning in Place (CIP) and Sterilization in Place (SIP). Accordingly, the present vessel may be internally provided with one or more nozzles for spraying cleaning and/or sterilization substances to the inner surface of the vessel.

In each instance of the vessel being provided with one or more parts which insert from the exterior (for example, a stirrer, a heating coil, a sparger, a probe, a sampler, a sterilization nozzle and so on), there should ideally be a seal keeping the vessel sealed around the entry point (usually, and most simply, a port or an opening in the vessel through which the peripheral part is inserted). The sealing assembly in a stirrer in a bioreactor is an important component that ensures proper agitation and prevents leakage through the stirrer shaft and helps maintain aseptic conditions during operation. Different types of sealing assemblies, including packed gland seals, mechanical seals, and magnetic drives, are utilized depending on the specific requirements of the fermentation or cultivation process.

### Bioreactor Apparatuses

The vessel itself may be provided as part of a larger bioreactor apparatus. Many such type of apparatus/bioreactors are known in the art; the present vessels can act as straight substitutes for existing vessels made from, for example, glass or stainless steel with no significant modification of the rest of the apparatus.

For example, the present vessels may be used as part of a stirred tank bioreactor, an externally agitated tank bioreactor, a packed bed bioreactor, a fluidised bed bioreactor or an airlift bioreactor.

The stirred tank bioreactor (STR) is a commonly used type of bioreactor in industrial applications. It consists of a cylindrical vessel with a central shaft controlled by a motor, supporting one or more stirrers or impellers. The combination of the sparger and impellers allows for efficient distribution of gases throughout the vessel, promoting optimal conditions for cell growth.

The STR offers several advantages in bioprocessing. It can be operated in several modes (such as batch, fed-batch, continuous, perfusion and so on). It is relatively easy to operate, resulting in lower labour costs, and the vessel is easy to clean, facilitating maintenance and preventing contamination. These attributes make it a practical choice for large-scale production. STRs offer a reliable and scalable solution for various bioprocesses in the pharmaceutical, chemical, and biotechnology industries.

The cylindrical vessel of a STR typically has a diameter-to-height ratio (aspect ratio) ranging from 1:1 to 1:5, with lower aspect ratios more typically used for animal cell culture while higher aspect ratios are favoured for fermentation of yeast and bacteria.

The number and positioning of impellers may depend on the aspect ratio of the vessel. Typically, the bottom impeller is located with an off-bottom clearance of about one-third of the tank's diameter. Additional impellers are typically spaced between one and two diameters apart, but configurations vary depending on specific mixing requirements. Moreover, the impeller types used vary depending on the application.

In animal cell culture vessels, a single, large-diameter, low-shear impeller such as a marine impeller is commonly employed. Gas is often introduced into the liquid through a sparger, either a perforated pipe ring with a slightly smaller diameter than the impeller or a single-hole sparger.

The externally agitated tank is a bioreactor that may have several shapes, often cylindrical, which is agitated by external mechanical means such as a rolling, rocking or a centrifugal platform, frame or stand.

In an airlift bioreactor the fluid volume is divided into two interconnected zones. One zone, known as the riser, is sparged with gas, while the other zone, called the downcomer, is not sparged with gas. The riser and the downcomer can be separate vertical pipes joined at the top and bottom to form an external circulation loop.

In some embodiments, an airlift bioreactor consists of two concentric vessels (one, smaller, inside and one, larger, outside). This forms the aforementioned two zones: one inside the inner vessel (usually acting as the riser, with the sparger positioned for example at the bottom) and one between the two vessels, i.e. outside the inner vessel but inside the outer vessel, (usually acting as the downcomer). The presently defined vessel may be used as one or both of these two vessels.

The gas-liquid dispersion in the riser flows upward due to its lower bulk density, while in the downcomer, it flows downward. This circulation is driven by the difference in gas holdup between the two zones. Airlift bioreactors are known for their energy efficiency and are particularly suitable for shear-sensitive cultures, making them ideal for the mass production of biopharmaceutical proteins derived from delicate animal cells.

Another advantage of airlift bioreactors is their simplicity of design. They do not contain any moving parts or agitators, making them easy to sterilize and maintain. They also have low energy requirements and are cost-effective compared to other types of bioreactors.

Overall, airlift bioreactors provide efficient gas-liquid mixing, favourable mass transfer characteristics, and low shear environments, making them valuable tools in various bioprocessing applications.

A packed bed bioreactor is a type of bioreactor that consists of a bed of solid particles within a vessel with cells either on or within the matrix of solids. The cells are immobilized on the solids which may be rigid or macroporous particles. The culture medium flows through the bed to provide the nutrients to the immobilized cells. Metabolites and products are released into the fluid and removed in the outflow.

Advantages of Packed Bed Reactors include their simple design, absence of moving parts, low operating cost, and continuous operation.

In a fluidized-bed bioreactor, the immobilized cells are placed at the bottom of the vessel, and the culture medium is pumped into the vessel, causing the bed to become fluidized. The cells are immobilized on small particles that move with the fluid, allowing for improved contact between the cells and the surrounding liquid. This design overcomes issues commonly associated with packed bed reactors, such as clogging, high liquid pressure drop, channelling, and bed compaction.

These bioreactors are particularly suitable for reactions involving fluid-suspended immobilized cells, and microbial flocs.

Bioreactors of the types described herein may be operated in a variety of manners (modes of operation) known to the skilled person. For example, they may be operated in batch; fed-batch; or continuous mode. In such continuous mode, there may be cell retention (perfusion) or may not be cell retention (chemostat).

In a batch mode, there is neither addition of fresh medium nor removal of spent medium from the culture (i.e. from the bioreactor) during a culture run. The nutrients are consumed along the time of culture while products from metabolism accumulate in the medium. Therefore, the physiological conditions during a batch culture are continuously changing. Cell growth is arrested when either nutrients are depleted or toxic metabolites achieve inhibiting levels, or both.

Vessels for reactor apparatuses for such an operating mode may include a medium/inoculum inlet to the main body. The reactor apparatus may include a fresh medium feed means, such as a reservoir in fluid communication with the medium/inoculum inlet. The reservoir may contain fresh cell culture medium to be added prior to the start of the culture run.

In a fed-batch mode, it is intended to maintain nutrient concentrations, often prolonging culture time and cell viability and thus increasing final product concentration. Fed batch operation usually allows both cell and product concentrations to accumulate at high levels. In such an operating mode, there is a feed supplied into the reactor during the culturing process. The feed can consist of a single nutrient or a concentrated set of nutrients in stoichiometric ratios, added in accordance with either empirical or complex models.

The objective in a fed-batch culture can be the maintenance of a high viable cell concentration or, if in the presence of an overflow metabolism, the achievement of energetically more efficient metabolic states. In the latter case, the strategy for feeding must consider both providing sufficient nutrients without any limitation and minimizing the production of undesired metabolites. This can be achieved by manipulating glucose and glutamine concentrations that, if kept at low levels, can shift metabolism to energetically more efficient states, thus reducing metabolite production and extending culture time and final titers. The set up of accurate on-line monitoring and control systems is very important in this type of culture, to add sufficient but not excessive nutrients for cell growth, in order to avoid the accumulation of non-desired subproducts.

Accordingly, vessels for reactor apparatuses for such an operation mode may advantageously include a medium/inoculum inlet to the main body. The reactor apparatus may include a fresh medium feed means, such as a reservoir in communication with the medium/inoculum inlet. The reservoir may suitably contain fresh cell culture medium or a mixture of specific nutrients such as glucose, glutamine, vitamins or amino acids.

For monitoring conditions within the vessel, and hence complete on-line monitoring of nutrients, metabolites and so on, the reactor apparatus may suitably include one or more online probes or sampling systems which can extract a sample from within the vessel and analyse it, normally external to the vessel. Depending on the result of the analysis, one or more nutrients can be fed into the vessel as needed. For example, the level of glutamine and/or glucose may be monitored or measured in this way.

In a continuous mode, the culturing process can be run as a chemostat culture, when the reactor is open to biomass removal along with the spent medium, or as perfusion, when a device for biomass retention is used, and only spent medium is removed from the bioreactor. In both cases there is a continuous inflow of fresh medium into the bioreactor. Accordingly, as with the types discussed above, vessels for reactor apparatuses for such operation modes include a feed inlet to the main body. The reactor apparatus may include a fresh medium feed means, such as a reservoir in fluid communication with the feed inlet. The reservoir may contain fresh cell culture medium. Vessels for reactor apparatuses for such operation modes also include a feed outlet for exiting the main body. There may be means, for example a pump, for actively withdrawing from the feed outlet.

In a perfusion mode, there is a continuous inflow of fresh medium to the bioreactor, but only spent medium is removed from the bioreactor, due to the inclusion of some device for biomass retention. This device can be situated inside or outside the bioreactor and can, amongst others, include spin filters, membrane filters, acoustic filters or centrifugation-based separators. The device is typically connected or situated such that it limits flow through the feed outlet. Alternatively, it may be connected such that it divides spent medium from cell culture, the cell culture being recycled into the main body (through the feed inlet or otherwise) and the spent medium moving on for further processing.

In a chemostat mode, there is a continuous inflow of fresh medium into the bioreactor and a continuous biomass removal together with the spent medium. Accordingly chemostat operation does not require the presence of a device for biomass retention as is mentioned for perfusion operation. Chemostat culture allows the study of a certain culture variable, maintaining all other variables constant.

Similarly, a wide variety of culture media and cell types can be utilised in the present vessels. Cells such as animal cells, bacteria, yeast, filamentous fungi, algae and plant cells are all suitable.

The vessels may be used to culture cells comprising proliferation (cells actively duplicating in exponential phase), production (cells in the stationary phase, after exponential growth) or differentiation phases.

Suitably, the vessels can be used to culture cells for cultivated meat, also known as cultured meat, lab grown meat, clean meat, cell based meat, cellular meat or for any cellular agriculture product.

The vessels may also be used to culture cells for cell and gene therapy.

The vessels may also be used to culture cells for any chemical, biochemical or biotechnological application, such as but not limited to chemical, environmental, energy, pharmaceutical and food applications.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

### Examples

### EXAMPLE 1

### Materials/Methods

### Vessel

A vessel of approximately 11.4 L capacity with external diameter of 184 mm and height of 448 mm, constructed of a composite material with a tank of plastic (high density polyethylene) reinforced with an outer shell of fibreglass (fibreglass with resin) for strength, having a 2.5 inch threaded top hole, was used.

### Sterility tests

Sterility tests on the stability of the tank structure were performed over 10 wet/steam autoclave cycles taking place over a three week period. Autoclave cycles involved a maintained temperature of at least 121 °C over a 20 minute period, with temperature logging showing that temperatures briefly reached 124 °C throughout runs.

### Bioreactor

An autoclavable threaded lid for the vessel was designed on Autodesk Fusion software and 3D printed in PA2200 Nylon. Threaded M6 ports were added to the vessel lid to accommodate fittings for a temperature sensor thermowell, dissolved oxygen probe, pH probe, sampling port, media addition/feed port, overlay gassing and gas exhaust port. An Eppendorf Dasgip 3L heating blanket was used to maintain the culture medium at the desired set-point throughout the culture period. The lid was printed with an 8.2 mm diameter central hole to accommodate the 8 mm stainless steel shaft used for the impeller system. A rubber O-ring was placed on the impeller shaft as a seal to maintain sterility. A 3-bladed, 45° pitched-blade Elephant Ear impeller was 3D printed in PA2200 Nylon to a diameter of 70 mm.

The impeller was designed with an 8 mm central hole on the impeller hub to fit onto the stainless steel impeller shaft. The impeller was stirred by an overhead variable-speed stirrer (Velp Scientifica).

### Biocompatibility tests

The autoclave-sterilised stirred (approx. 240 rpm) vessel was filled with 7 L of proprietary protein-free culture medium which was incubated until the desired temperature (41 °C), DO (dissolved oxygen) level (30%) and pH (7.2) stabilised. The vessel was then inoculated with a suspension of spontaneously immortalized chicken fibroblast cells at a final viable cell concentration (VCC) of approximately 1.1 × 10⁵ cells/mL. The cell viability upon inoculation was 94%. Viable cell concentration (VCC) and cell viability were monitored daily using a Countess^{™} Automated Cell Counter (Fisher Scientific). Analytes including glucose and lactate levels were monitored daily using a YSI 2900 Analyser (YSI/Xylem), while pH and osmolality (measured using a Gonotec Osmomat 3000) were measured across the culture period for comparison with prior data.

### Results

### Sterility tests

After each wet/steam autoclave cycle, a detailed visual inspection was made to the inside and outside of the vessel, with the conclusion that even after 10 wet/steam autoclave cycles, the integrity and shape of the vessel structure was maintained.

These results are of great significance since they prove that the material can be sterilised by wet steam / high temperature cycles, making it viable to be used repetitively in cell culture under aseptic conditions.

### Biocompatibility tests

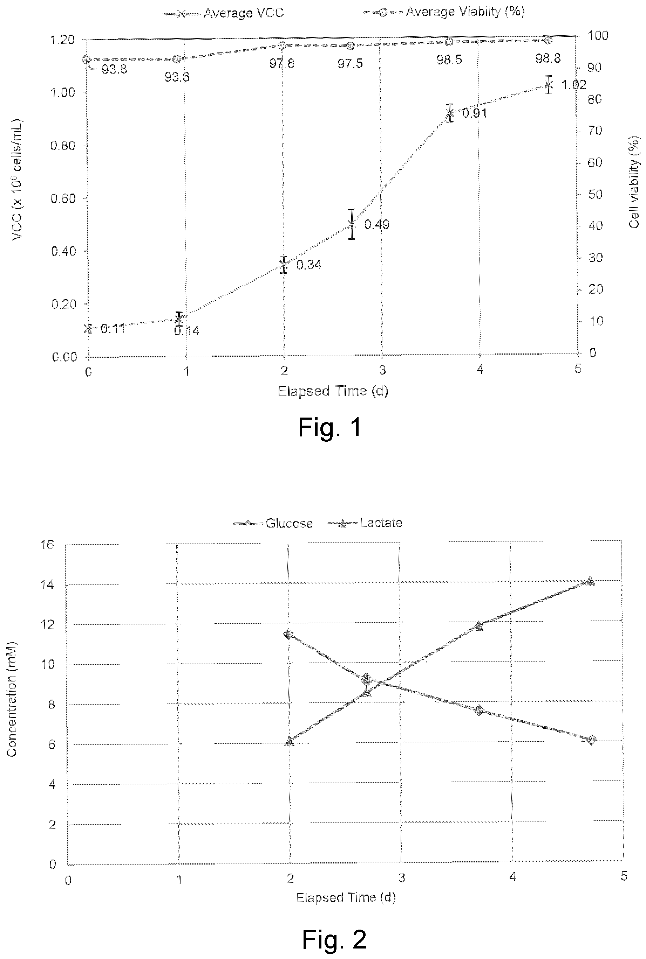

The culture was run in batch mode until cell concentration started to plateau, which occurred approximately by day 5. The maximum cell concentration achieved was over 1 million cells/ml and the cell viabilities were always over 93% (see **Fig. 1**, plotting average viable cell concentration (VCC) and average cell viability (%) overtime).

The cell metabolism, measured by glucose consumption and lactate production, was normal (see **Fig. 2**, plotting glucose and lactate concentrations overtime).

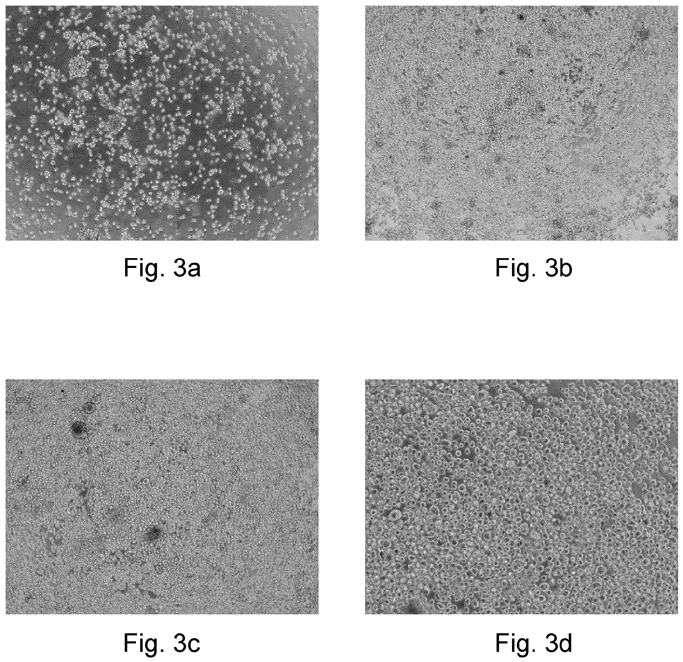

The cell morphology in suspension was normal, showing healthy cells (see **Fig. 3**; with **Fig. 3a** showing a sample of the cell culture on day 1 at 10x magnification; **Fig. 3b** showing a sample of the cell culture on day 3 at 10x magnification; **Fig. 3c** showing a sample of the cell culture on day 4 at 10x magnification; and **Fig. 3d** showing a sample of the cell culture on day 4 at 20x magnification).

After the run, no cell adhesion to the internal surface was observed, meaning that the material is not only biocompatible but also it does not promote cell adhesion, which in animal cells, is quite often seen in several surfaces, including those made of plastic.

### Conclusion

Taken together, the results obtained show that animal cells can grow normally in the present vessels, with normal cell densities, viabilities and morphologies being observed. This clearly proves that the material of the vessel is biocompatible. Moreover, no adhesion of cells to the internal surface was observed, which is positive in terms of the use of the vessel in longer runs and at larger scales.

The significance of these results is enormous, since they validate the use of these vessels in cell culture, being an affordable alternative to the expensive materials being used for decades. The implications in terms of the investment needed are very positive, since significant reductions in the capital expenditure (CAPEX) are now possible, making the depreciation component of the cost structure much lower, contributing to the economic viability of lower added-value biologic products, such as but not limited to cultivated meat products or cellular agriculture products in general.

## Claims

1. Use of a vessel, comprising a rigid tank comprising a plastic or first cured resin material which is biocompatible and which can withstand steam sterilization, as a bioreactor.

2. Use according to claim 1, wherein the vessel further comprises an outer shell of reinforcing fibres impregnated with a second cured resin, the outer shell at least partially encompassing the tank.

3. Use according to claim 1 or claim 2, wherein the rigid tank is formed from a material selected from polyethylene, polystyrene, polycarbonate, polypropylene, polyvinyl chloride and polyurethane; preferably wherein rigid tank is formed from a material selected from polyethylene and polypropylene; more preferably wherein the rigid tank is formed from polyethylene for example high density polyethylene.

4. Use according to claim 2 or claim 3, wherein the reinforcing fibres are woven.

5. Use according to any one of claims 2-4, wherein the reinforcing fibres are preimpregnated with a resin which is then cured to form the outer shell.

6. Use according to any one of claims 2-5, wherein the second cured resin is an epoxy resin, a phenolic resin, a bismaleimide resin, or a polyimide resin; preferably wherein the second cured resin is an epoxy resin.

7. Use according to any one of claims 2-6, wherein the reinforcing fibres are carbon fibres, fibreglass fibres or aramid fibres.

8. Use according to any one of the preceding claims, wherein the vessel has baffles positioned within the tank, optionally attached to the walls of the tank.

9. Use according to any one of the preceding claims, wherein the use is as a stirred tank, externally agitated tank, packed bed, fluidised bed or airlift bioreactor.

10. Use according to any one of the preceding claims, wherein the use is as a bioreactor in a batch, fed-batch, continuous with cell retention, or continuous without cell retention mode of operation.

11. Use according to any one of the preceding claims, wherein the use is as a bioreactor for culturing animal cells, bacteria, yeast, filamentous fungi, algae or plant cells.

12. A bioreactor apparatus, comprising a vessel, the vessel comprising a rigid tank comprising a plastic or first cured resin material which is biocompatible and which can withstand steam sterilization and optionally further comprising an outer shell of reinforcing fibres impregnated with a second cured resin;
the apparatus further comprising at least one of:
(a) agitation means, for agitating the content of the vessel;
(b) temperature control means, for controlling the temperature of the content of the vessel;
(c) fluid introduction means, for introducing fluid, suitably liquid, into the vessel; and
(d) sparging means, for introducing gas into the vessel.

13. A bioreactor apparatus according to claim 12, wherein the agitation means is a stirrer positioned within the vessel, or a rocking, rolling or centrifugally moveable platform positioned external to the vessel.

14. A bioreactor apparatus according to claim 12 or claim 13, wherein the temperature control means is a heated jacket positioned around the outside of the main body, a double wall water jacket, a coil jacket or an internal heating coil.

15. A bioreactor apparatus according to any one of claims 12-14, wherein the fluid introduction means comprises a pipe connected to an aperture in the main body, the pipe having a valve to control flow of fluid through the pipe into the main body.
